# EUROPEAN PATENT APPLICATION

(11) **EP 1 795 185 A2**
(43) Date of publication of application: **13.06.2007**
(21) Application number: 06256206.1
(22) Date of filing: 06.12.2006
(51) Int. Cl.: A61K 9/16, A61K 9/51, A61K 9/00

(54) **Nano- and/or micro-particulate formulations for local injection-based treatment of vascular diseases**

(30) Priority: 07.12.2005 US 296101
(71) Applicant: Cordis Corporation, Miami Lakes, FL 33014 (US)
(72) Inventor: Zhao, Jonathon, Belle Mead NJ 08502 (US)
(74) Representative: Belcher, Simon James

(57) **Abstract**

The present invention relates to nano- and/or micro-particulate formulations that can be locally injected into arterial walls at or near target sites to achieve a prolonged and sufficiently high local concentration of at least one pharmacologically active agent for treatment of vascular diseases, such as, for example, restenosis, vulnerable plaque, aneurysm, and stroke. Specifically, each formulation comprises biocompatible and biodegradable nano-particles and/or micro-particles loaded with the pharmacologically active agent and d-alpha-tocopheryl polyethylene glycol 1000 succinate (vitamin E TPGS). The formulations can be formed by a solvent evaporation/extraction process or a supercritical CO₂ extraction process that uses vitamin E TPGS as an emulsifier. Further, vitamin E TPGS can be used as a stabilizer for the pharmacologically active agent in the final drug formulation, as well as a release modulation to control release of the pharmacologically active agent.

## Description

The present invention relates to nano- and micro-particulate formulations for the treatment of vascular diseases, such as restenosis, vulnerable plaque, aneurysm, and/or stroke. More specifically, the present invention relates to nano- and micro-particulate formulations that can be locally injected into target sites in the arterial walls to effectuate sustained local delivery of pharmacologically active agents at sufficiently high local concentrations for treatment of the respective vascular diseases.

The recently developed drug-eluting stents, such as stents sold under the trade names of Cypher and Taxus, have demonstrated outstanding results for local drug delivery. For example, the Cypher stent, which has a rapamycin-containing coating, has consistently demonstrated superior efficacy against restenosis after its implantation, in comparison with uncoated stents. The Taxus stent, which has a paclitaxel-containing coating, also demonstrated potent anti-restenotic effects, despite a relatively short duration of drug release from the stent coating. These drug-eluting stents have drug-containing coatings and can be implanted into the body to release potent anti-inflammatory and anti-neoplastic agents, such as rapamycin and/or paclitaxel, in a controlled manner to the adjacent tissue.

Local delivery of these anti-inflammatory and anti-neoplastic agents by the implanted stents does not result in any significant increase of the overall drug concentration in the body, thereby reducing the potential toxic effect of the drugs to other tissues or organs. Further, the high local concentration and prolonged tissue retention of the anti-inflammatory and anti-neoplastic agents achieved by the implanted stents ensure complete elimination or effective reduction of neointimal growth after an angioplasty procedure.

However, a less invasive approach for local drug delivery that does not involve stent implantation may be desirable in certain clinical situations, such as operations involving bifurcation junction or small arteries, or in the case of restenosis after previously placement of stents.

There is therefore a continuing need to provide improved and less invasive devices and methods for local drug delivery.

The present invention in one aspect relates to a formulation comprising biocompatible and biodegradable nano-particles and/or micro-particles loaded with at least one pharmacologically active agent efficacious for treating vascular diseases, wherein the formulation further comprises d-alpha-tocopheryl polyethylene glycol 1000 succinate at a concentration ranging from about 0.01 wt % to about 20 wt % of the total weight of the formulation.

The term "nano-particles" or "micro-particles" is used throughout the present invention to denote carrier structures that are biocompatible and have sufficient resistance to chemical and/or physical destruction by the environment of use such that a sufficient amount of the nano-particles and/or micro-particles remain substantially intact after injection into a target site in the arterial wall. Typically, the nano-particles of the present invention have sizes ranging from about 1 nm to about 1000 nm, with sizes from about 100 nm to about 500 nm being more preferred. The micro-particles of the present invention have sizes ranging from about 1 µm to about 1000 µm, with sizes from about 10 µm to about 200 µm being more preferred. The pharmacologically active agent as described hereinabove is loaded within and/or on the surfaces of the nano-particles and/or micro-particles.

The term "biocompatible" as used herein refers to any material, composition, structure, or article that have essentially no toxic or injurious impact on the living tissues or living systems which the material, composition, structure, or article is in contact with and produce essentially no immunological response in such living tissues or living systems. More particularly, the material, composition, structure, or article has essentially no adverse impact on the growth and any other desired characteristics of the cells of the living tissues or living systems that are in contact with the material, composition, structure, or article. Generally, the methods for testing the biocompatibility of a material, composition, structure, or article is well known in the art.

The term "biodegradable" as used herein refers to any material, composition, structure, or article that will degrade over time by action of enzymes, by hydrolytic reaction, and/or by similar mechanisms in the body of a living organism.

In another aspect, the present invention relates to a method for forming a nano- and/or micro-particulate formulation, comprising encapsulating at least one pharmacologically active agent efficacious for treating vascular diseases into biocompatible and biodegradable nano-particles and/or micro-particles by a solvent evaporation/extraction process or a supercritical CO₂ dilution and extraction process, wherein d-alpha-tocopheryl polyethylene glycol 1000 succinate is used during the encapsulation process, and wherein the resulting nano- and/or micro-particulate formulation comprises d-alpha-tocopheryl polyethylene glycol 1000 succinate at a concentration ranging from about 0.01 wt % to about 20 wt % of the total weight of the formulation.

The present invention can be used in a method for treating vascular diseases, comprising:
forming a nano- and/or micro-particulate formulation comprising biocompatible and biodegradable nano-particles and/or micro-particles loaded with at least one pharmacologically active agent efficacious for treating vascular diseases, wherein the formulation further comprises d-alpha-tocopheryl polyethylene glycol 1000 succinate at a concentration ranging from about 0.01 wt % to about 20 wt % of the total weight of the formulation; and
locally injecting the formulation into an arterial wall at a target site, or in the vicinity thereof, wherein the formulation release the at least one pharmacologically active agent in a sustained and controlled manner to achieve a prolonged and sufficiently high local concentration of the at least one pharmacologically active agent at the target site for treating the vascular diseases.

A local concentration of the at least one pharmacologically active agent higher than 1 ng per mg of tissue may be achieved at or near the target site and may be sustained for at least 1 week.

An injection catheter may be used for local injection of the formulation into the arterial wall at or near the target site.

The local injection may be carried out with guidance from intravascular ultrasound (IVUS) or angiography.

In a further aspect of the invention, at least two different pharmacologically active compounds are separately encapsulated into nano- and/or micro-particles to form at least two different portions of nano- and/or micro-particles, consistent with the descriptions hereinabove, which are then blended before use at a predetermined ratio. Specifically, the predetermined ratio of the at least two portions of nano- and/or micro-particles is calculated based on a group of variables, such as the LogP value of each compound, expected duration of drug release, and the inherent potency of each compound, so as to achieve optimal clinical results.

The present invention can also be used in a method for treating vascular disease, comprising:
forming a first portion of biocompatible and biodegradable nano- and/or micro-particles that are loaded with a first pharmacologically active agent and d-alpha-tocopheryl polyethylene glycol 1000 succinate, wherein d-alpha-tocopheryl polyethylene glycol 1000 succinate is present at a first concentration ranging from about 0.01 wt % to about 20 wt % of the total weight of said first portion of nano- and/or micro-particles;
forming a second portion of biocompatible and biodegradable nano-particles and/or micro-particles loaded with a second, different pharmacologically active agent and d-alpha-tocopheryl polyethylene glycol 1000 succinate, wherein d-alpha-tocopheryl polyethylene glycol 1000 succinate is present at a second concentration ranging from about 0.01 wt % to about 20 wt % of the total weight of said second portion of nano- and/or micro-particles;
combining the first and second portions of biocompatible and biodegradable nano-particles and/or micro-particles at a predetermined ratio to form a composite formulation;
locally injecting the composite formulation into an arterial wall at or near at least one target site, wherein the formulation release the first and second pharmacologically active agents in a sustained and controlled manner.

The predetermined ratio may be calculated based on one or more variables selected from the group consisting of LogP values of the first and second pharmacologically active agents, expected duration of release, and inherent potencies of the first and second pharmacologically active agents.

Other aspects, features and advantages of the invention will be more fully apparent from the ensuing disclosure and appended claims.

In the following description, numerous specific details are set forth, such as particular materials, compositions, formula, structures, devices, and methods for fabricating or using same, in order to provide a thorough understanding of the present invention. However, it will be appreciated by one of ordinary skill in the art that the invention may be practiced without these specific details. In other instances, well-known materials, structures or processing steps have not been described in detail in order to avoid obscuring the invention.

The present invention provides micro- and/or nano-particulate formulations for local injection into target sites in the arterial walls for controlled and sustained delivery of pharmacologically active agents. Prolonged and sufficiently high local concentrations (typically greater than 1 ng per mg of tissue) of the pharmacologically active agents can be achieved by the present invention for effective treatment of vascular diseases, such as, for example, restenosis, vulnerable plaque, aneurysm, and/or stroke. For example, for treatment of restenosis post the angioplasty procedure, it is preferred to have drug concentrations higher than 4-6 ng per mg of tissue at 24 hours post administration and at least 1 ng per mg of tissue for the next 1 month.

The micro- and/or nano-particulate formations of the present invention each comprises biocompatible and biodegradable nano-particles and/or micro-particles loaded with at least one pharmacologically active agent efficacious for treating vascular diseases.

More importantly, d-alpha-tocopheryl polyethylene glycol 100 succinate, which is also referred to as vitamin E TPGS, is provided in the micro- and/or nano-particulate formations of the present invention. Vitamin E TPGS is a water-soluble derivative of natural vitamin E, which has the following chemical formula: 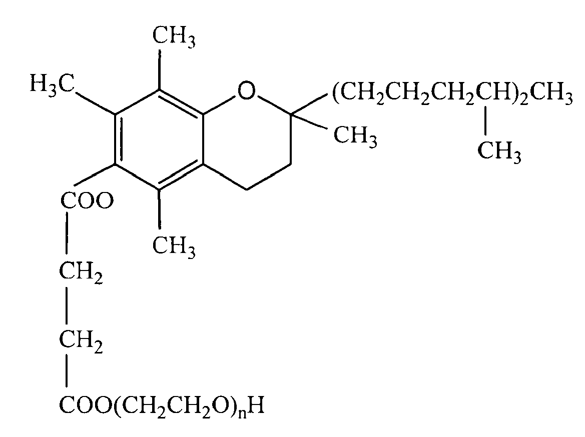 Vitamin E TPGS performs several important functions in the micro- and/or nano-particulate formations of the present invention.

First, the micro- and/or nano-particulate formations are formed by a solvent evaporation/extraction process, during which vitamin E TPGS is used as an emulsifier to facilitate encapsulation or loading of the pharmacologically active agent into the nano-particles and/or micro-particles. The chemical structure of vitamin E TPGS comprises both liphophilic and hydrophilic functional groups, resulting in its amphiphilic properties. Therefore, vitamin E TPGS can be used as an emulsifier to enhance the drug encapsulation or loading efficiency (up to 100%) in the micro-particles and/or nano-particles. Further, vitamin E TPGS can function as a surfactant to reduce aggregation/agglomeration of the suspended micro-particles and/or nano-particles in an already-formed formulation and allow easier passage of the formulation through the injection needle of a significantly narrow diameter (typically less than 70 µm).

Vitamin E TPGS can also function as a stabilizer to protect the pharmacologically active agent against oxidative degradation. Similar to natural vitamin E compounds, vitamin E TPGS exhibits active antioxidant functionality. Therefore, it can protect the pharmacologically active agent against potential oxidative degradation and prolong the shelf life of the formulation. Further, it may also play a role in reducing the potential damage to the healthy local tissues from the released pharmacologically active agent or the byproducts formed by degradation of the polymeric matrix.

Vitamin E TPGS can further function as a release modulator to facilitate the uptake of water into the target site (i.e., the sites in the arterial walls where the formulation of the present invention is injected), to slow down the degradation of the polymeric matrix of the micro- and/or nano-particles, and to thereby control the release rate of the pharmacologically active agent from the micro- and/or nano-particles into the surrounding tissue near a target site after injection of the micro- and/or nano-particulate formulation into the target site. Slow degradation of the polymeric matrix is important since the degradation byproducts, when reaching certain concentrations, may be toxic to the local tissue. Vitamin E TPGS can therefore be combined with various biocompatible and biodegradable polymers, as described in greater detail hereinafter, to achieve desired polymeric matrix degradation rate as well as drug release kinetics.

When used at a relatively low concentration (e.g., from about 0.01 wt % to about 1 wt % of the total solid weight of the polymeric matrix, the pharmacologically active agent, vitamin E TPGS, and any other additives), vitamin E TPGS functions as the emulsifier and surfactant only to enhance the encapsulation or loading efficiency of the pharmacologically active agent.

When used as a relatively high concentration (e.g., from about 1 wt % to about 20 wt %), vitamin E TPGS also functions as the stabilizer and the release modulator to help achieving optimal pharmacokinetic (PK) profile of the pharmacologically active agent, especially when the pharmacologically active agent is water-insoluble (such as rapamycin and paclitaxel). The relatively high concentration of vitamin E TPGS in the formulation also changes the formulation density and reduces the need for more complicated injection devices.

Preferably, the at least one pharmacologically active agent used in the present invention is a potent anti-inflammatory and anti-neoplastic agent, such as, for example, rapamycin, rapamycin ester, everolimus, zotarolimus (formerly known as ABT-578), biolimus, tacrolimus, pimecrolimus, and wortmannin, taxanes such as paclitaxel, docetaxel, camptothecin, estradiol, Panzem, morphine, epothilone, matrix metalloproteinase (MMP) inhibitor such as tetracycline, and their associated derivatives and analogs. Such an anti-inflammatory and anti-neoplastic agent can effectively eliminate neointimal growth post an angioplasty procedure and therefore can be used to prevent or treat restenosis-induced vascular diseases, such as restenosis, vulnerable plaque, aneurysm, and/or stroke. Any pharmacologically active compound with a relatively low water solubility can be encapsulated into nano- and or micro-particles to form the nano/micro-particulate formations of the present invention. Large molecular weight entities, such as proteins, polypeptides, plasmids, DNAs, RNAs, ribozymes, DNases, siRNAs, antisense drugs, etc., can all be formulated according to the present invention.

One particular important aspect of the present invention is the use of vitamin E TPGS at relatively high concentrations in the final formulations to help enhance the stability of the therapeutic agents in the formulations. Many important therapeutic agents efficacious for vascular applications, such as drugs in the macrolide family, contain unsaturated double bonds that are susceptible to oxidative degradation. Vitamin E TPGS has antioxidant functionalities and can therefore be used to enhance the stability of macrolide drugs, such as rapamycin, during the storage. Vitamin E TPGS further function as an emulsifier to facilitate encapsulation of the drugs into the nano- and/or micro-particles. Moreover, vitamin E TPGS as used in the present application does not need to be removed after formation of the drug-encapsulating nano- and or micro-particles, as in the cases of other emulsifiers, such as polyvinyl alcohol (PVA), Tween 20, and Tween 80. Instead, vitamin E TPGS is kept in the final nano- and/or micro-particulate formulations of the present invention to stabilize the drugs contained therein.

In a preferred but not necessary embodiment of the present invention, the nano- and/or micro-particulate formulation comprises at least rapamycin. Rapamycin, also referred to as sirolimus, is a macrocyclic triene antibiotic produced by Streptomyces hygroscopicus as disclosed in US-3,929,992. It has been found that rapamycin, among other things, inhibits the proliferation of vascular smooth muscle cells *in vivo.* Accordingly, rapamycin may be utilized in treating intimal smooth muscle cell hyperplasia, restenosis, and vascular occlusion in a mammal, particularly following either biologically or mechanically mediated vascular injury, or under conditions that would predispose a mammal to suffering such a vascular injury. Rapamycin functions to inhibit smooth muscle cell proliferation and does not interfere with the re-endothelialization of the vessel walls. Rapamycin reduces vascular hyperplasia by antagonizing smooth muscle proliferation in response to mitogenic signals that are released during an angioplasty-induced injury. Inhibition of growth factor and cytokine mediated smooth muscle proliferation at the late G1 phase of the cell cycle is believed to be the domain mechanism of action of rapamycin. However, rapamycin is also known to prevent T-cell proliferation and differentiation when administered systematically, and it therefore can be used as an immunosuppressant for preventing graft rejection.

In a more preferred embodiment of the present invention, the micro- and/or nano-particulate formation comprises two or more pharmacologically active agents of different pharmacologically mechanisms. For example, the formulation may comprise rapamycin and estradiol for treatment of restenosis and vulnerable plaque. Other drug combinations, such as rapamycin with tetracycline or rapamycin with a PI3 kinase such as wortmannin or its derivative PX 867, can be used in conjunction in a similar manner.

In another preferred embodiment of the present invention, the micro- and/or nano-particulate formation comprises at least two or more portions of biocompatible and biodegradable nano-particles and/or micro-particles loaded with the pharmacologically active agent and vitamin E TPGS at different loading doses. For example, the micro- and/or nano-particulate formation may comprise at least a first portion of nano-particles and/or micro-particles loaded with the pharmacologically active agent at a first loading dose and vitamin E TPGS at a second loading dose, and a second portion of nano-particles and/or micro-particles loaded with the pharmacologically active agent at a third loading dose and vitamin E TPGS at a fourth loading dose, while the first loading dose is greater than the third loading dose, and the second loading dose is greater than the fourth loading dose. More specifically, the first portion of nano-particles and/or micro-particles are loaded with vitamin E TPGS at a higher loading dose (e.g., from about 1 wt % to about 20 wt %), and the second portion of nano-particles and/or micro-particles are loaded with vitamin E TPGS at a lower loading dose (e.g., from about 0.01 wt % to about 1 wt %). Such a nano- and/or micro-particulate formation can be used to achieve a desired PK profile of the local concentration of the pharmacologically active agent, which, for example, is characterized by an initial, short high local concentration (e.g., greater than 5 ng/mg of tissue) of the pharmacologically active agent for the first day, followed by prolonged low local concentration (e.g., about 1 ng/mg of tissue) of the pharmacologically active agent for the next 30 days.

The at least one pharmacologically active agent as described hereinabove is encapsulated into biocompatible and biodegradable nano-particles and/or micro-particles that are formed by at least one biocompatible and biodegradable polymeric material, which function as a carrier matrix to provide support for the pharmacologically active agent as well to control the release thereof.

The term "polymer" or "polymeric" as used herein refers to any material, composition, structure, or article that comprises one or more polymers, which can be homopolymers, copolymers, or polymer blends.

The biocompatible and biodegradable polymeric material of the present invention can be either a homopolymer, a copolymer, or a polymer blend that is capable of releasing the pharmacologically active agent into at least one target site in the arterial walls in a controlled and sustained manner after local injection. Suitable polymeric materials that can be used in the present invention include, but are not limited to: polylactic acid (PLA), polyglycolid acid (PGA), copolymers of lactic acid and glycolic acid (PLGA), polycaprolactone, polyphosphoester, polyorthoester, poly(hydroxy butyrate), poly(diaxanone), poly(hydroxy valerate), poly(hydroxy butyrate-co-valerate), poly(glycolide-co-trimethylene carbonate), polyanhydrides, polyphosphoester, poly(ester-amide), polyphosphoeser, polyphosphazene, poly(phosphoester-urethane), poly(amino acids), polycyanoacrylates, biopolymeric molecules such as fibrin, fibrinogen, cellulose, starch, collagen and hyaluronic acid, and mixtures and copolymers of the foregoing.

Preferably, the biocompatible and biodegradable polymeric material of the present invention is selected from the group consisting of PLA, PGA, PLGA, and mixtures thereof. More preferably, the biocompatible and biodegradable polymeric material of the present invention comprises the PLGA copolymer. The PLA, PGA, or PLGA polymers may be any of D-, Land D-/L- configuration. It is preferred that biocompatible and biodegradable polymeric material of the present invention comprises PLA, PGA, or PLGA polymers with a ratio of D-/L- configuration (mol %) ranging from about 75/25 to about 25/75, more preferably from about 60/40 to about 30/70.

The degradation process of the above-mentioned polymers, either *in vivo* or *in vitro,* is affected by several factors, including preparation method, molecular weight, composition, chemical structure, size, shape, crystallinity, surface morphology, hydrophobicity, glass transition temperature, site of loading, physicochemical parameters in the surrounding environment (such as pH, temperature, and ionic strength), and mechanism of hydrolysis. Specifically, the degradation behavior of nano-particles and/or micro-particles depends on hydrophilicity of the polymer used for forming such particles: the more hydrophilic the polymer, the more rapid its degradation. The hydrophilicity of the polymer is influenced by the ratio of crystalline to amorphous regions, which in turn is determined by the polymeric composition and monomer stereochemistry. For example, PLGA copolymer prepared from L-PLA and PGA are crystalline copolymers, while those from D-, L-PLA and PGA are amorphous in nature. Lactic acid, being more hydrophobic than glycolic acid, makes lactic acid-rich PLGA copolymers less hydrophilic and subsequently slows down the degradation process.

In general, the degradation time will be shorter for low molecular weight, more hydrophilic, more amorphous polymers and copolymers with higher content of glycolic acid. In accordance with these variables, the *in vivo* degradation of the D-, L-PLGA copolymer may vary from a few weeks to more than 1 year.

Further, as mentioned hereinabove, the concentration of vitamin E TPGS loaded into the nano-particles and/or micro-particles impacts the biodegradation rate of such particles. Therefore, different polymeric compositions can be combined with different concentrations of vitamin E TPGS to achieve desired *in vivo* degradation rate of the nano-particles and/or micro-particles.

The *in vivo* biodegradation rate of the polymeric nano-particles and/or micro-particles of the present invention is important because it determines the rate and mechanism of release of the pharmacologically active agent carried by such nano-particles and/or micro-particles. The release of the pharmacologically active agent from the polymeric matrix of the nano-particles and/or micro-particles is biphasic, i.e., including an initial phase of diffusion through the polymeric matrix and a subsequent phase of both diffusion of the pharmacologically active agent and the degradation of the polymeric matrix itself.

Therefore, by controlling the *in vivo* degradation rate of the polymeric nano-particles and/or micro-particles that carry the pharmacologically active agent, the release rate of the pharmacologically active agent can be readily adjusted. Preferably, the nano-particles and/or micro-particles release the pharmacologically active agent for a prolonged period of time, e.g., ranging from about 1 week to about 1 year. More preferably, the nano- and/or micro-particulate formulation of the present invention contains nano-particles and/or micro-particles that release the pharmacologically active agent at different rates, so that the overall release pattern of the pharmacologically active agent can be readily adopted for specific applications.

The nano-particles and/or micro-particles of the present invention can be readily formed by a solvent evaporation/extraction process, a supercritical CO₂ dilution/extraction process, or any other known methods for forming nano-particles and/or micro-particles.

For example, the biocompatible and biodegradable polymeric material and the pharmacologically active agent are dissolved in one or more organic solvents to form an organic phase mixture. The organic phase mixture is then slowly added into an aqueous solution that contains vitamin E TPGS with or without other additives, while sufficient agitation is applied either by stirring or sonication, or both. The oil/water emulsion so formed can then be gently stirred at room temperature overnight to allow the organic solvent(s) to evaporate, followed by freeze-drying to obtain polymeric nano-particles and/or micro-particles loaded with pharmacologically active agent. The processing steps involved in a solvent evaporation/extraction process are well known in the art, with the exception of the use of the vitamin E TPGS to achieve desired loading efficiency, particle size, and enhanced stability of the incorporated drug, so they are not described in detail herein in order to avoid obscuring the invention. Preferably but not necessarily, a relatively high concentration of vitamin E TPGS (i.e., 1 wt% to about 20%) and a safe solvent, such as ethyl acetate (EA), are used during the solvent evaporation/extraction. Further, vitamin E TPGS is not removed after the formulation process, but is maintained in the final formulation. Moreover, the particle size of the resulting nano- and or micro-particulate formulation can be readily adjusted by changing the vitamin E TPGS concentration and/or the agitation speed used during the solvent evaporation/dilution process. The higher the concentration of vitamin E TPGS and the faster the agitation speed, the smaller the particle size.

The micro- and/or nano-particle formulations of the present invention can be locally delivered into one or more target sites in the arterial walls by injection. Under the guidance of intravascular ultrasound (IVUS) or angiography, the micro- and/or nano-particle formulations can be directly injected into and deposited at the most affected spots in the arterial walls, instead of being released into the blood stream.

Such a direct injection of the drug-containing micro- and/or nano-particle formulations into the arterial walls can achieve a relatively high local concentration of the pharmacologically active agents in the arterial tissues, without significantly increasing the overall systematic concentration of the pharmacologically active agents in the blood stream. Sustained and controlled release of the pharmacologically active agents from the deposited drug-containing micro- and/or nano-particles enables prolonged permeation of the pharmacologically active agents into the surrounding arterial tissue. Further, by encapsulating the pharmacologically active agents into the micro- and/or nano-particles, the local tissues are shielded from the potential toxic effect of the pharmacologically active agents when provided in direct contact with the tissues at high concentrations.

The micro- and/or nano-particle formulations of the present invention can be readily delivered to local arterial tissues by injection catheters, such as weeping balloon catheters or micro-needle injectors. Such formulations provide viable treatment for vulnerable plaques (VP) and prophylactic treatment of stroke.

While specific embodiments of the present invention are described and illustrated hereinabove, it is clear that a person ordinarily skilled in the art can readily modify such specific embodiments consistent with the descriptions provided herein. It should therefore be recognized that the present invention is not limited to the specific embodiments illustrated hereinabove, but rather extends in utility to any other modification, variation, application, and embodiment, and accordingly all such other modifications, variations, applications, and embodiments are to be regarded as being within the spirit and scope of the invention.

## Claims

1. A formulation comprising biocompatible and biodegradable nano-particles and/or micro-particles loaded with at least one pharmacologically active agent efficacious for treating vascular diseases, wherein said formulation further comprises d-alpha-tocopheryl polyethylene glycol 1000 succinate at a concentration ranging from about 0.01 wt % to about 20 wt % of the total weight of said formulation.

2. The formulation of claim 1, comprising d-alpha-tocopheryl polyethylene glycol 1000 succinate at a concentration ranging from about 0.01 wt % to about 1 wt % of the total weight of said formulation.

3. The formulation of claim 1, comprising d-alpha-tocopheryl polyethylene glycol 1000 succinate at a concentration ranging from about 1 wt % to about 20 wt % of the total weight of said formulation.

4. The formulation of claim 1, wherein the at least one pharmacologically active agent is selected from the group consisting of rapamycin, rapamycin ester, everolimus, zotarolimus, biolimus, tacrolimus, pimecrolimus, PX 867, wortmannin, taxanes, paclitaxel, docetaxel, camptothecin, estradiol, Panzem, morphine, epothilone, tetracycline, and derivatives and analogs thereof.

5. The formulation of claim 1, comprising two or more pharmacologically active agents of different pharmacological mechanisms.

6. The formulation of claim 5, comprising at least rapamycin and estradiol.

7. The formulation of claim 5, comprising at least rapamycin and tetracycline.

8. The formulation of claim 5, comprising at least rapamycin and PX 867.

9. The formulation of claim 1, comprising at least two or more portions of biocompatible and biodegradable nano-particles and/or micro-particles loaded with the at least one pharmacologically active agent and d-alpha-tocopheryl polyethylene glycol 1000 succinate at different loading doses.

10. The formulation of claim 9, comprising at least a first portion of biocompatible and biodegradable nano-particles and/or micro-particles loaded with the at least one pharmacologically active agent at a first loading dose and d-alpha-tocopheryl polyethylene glycol 1000 succinate at a second loading dose, and a second portion of biocompatible and biodegradable nano-particles and/or micro-particles loaded with the at least one pharmacologically active agent at a third loading dose and d-alpha-tocopheryl polyethylene glycol 1000 succinate at a fourth loading dose, wherein the first loading dose is greater than the third loading dose, and wherein the second loading dose is greater than the fourth loading dose.

11. The formulation of claim 10, wherein the second loading dose ranges from about 1 wt % to about 20 wt % of the total weight of the first portion, and wherein the fourth loading dose ranges from about 0.01 wt % to about 1 wt % of the total weight of the second portion.

12. The formulation of claim 1, wherein the biocompatible and biodegradable nano-particles and/or micro-particles comprise at least one biocompatible and biodegradable polymeric material comprising a homopolymer, a copolymer, or a polymer blend that is capable of releasing the at least one pharmacologically active agent into at least one target site in the arterial walls in a controlled and sustained manner after local injection.

13. The formulation of claim 12, wherein the at least one biocompatible and biodegradable polymeric material is selected from the group consisting of polylactic acid (PLA), polyglycolid acid (PGA), copolymers of lactic acid and glycolic acid (PLGA), poly(ester amide), polycaprolactone, polyphosphoester, polyorthoester, poly(hydroxy butyrate), poly(diaxanone), poly(hydroxy valerate), poly(hydroxy butyrate-co-valerate), poly(glycolide-co-trimethylene carbonate), polyanhydrides, poly(phosphoester-urethane), poly(amino acids), polycyanoacrylates, fibrin, fibrinogen, cellulose, starch, collagen, hyaluronic acid, and copolymers and mixtures thereof.

14. The formulation of claim 12, wherein the at least one biocompatible and biodegradable polymeric material is selected from the group consisting of PLLA, PGA, PLGA, and mixtures thereof.

15. The formulation of claim 1, comprising biocompatible and biodegradable nano-particles having a particle size ranging from about 1 nm to about 1000 nm.

16. The formulation of claim 1, comprising biocompatible and biodegradable micro-particles having a particle size ranging from about 1 µm to about 1000µm.

17. A method for forming a nano- and/or micro-particulate formulation, comprising encapsulating at least one pharmacologically active agent efficacious for treating vascular diseases into biocompatible and biodegradable nano-particles and/or micro-particles by a solvent evaporation/extraction process or a supercritical CO₂ extraction process, wherein d-alpha-tocopheryl polyethylene glycol 1000 succinate is used during the encapsulation process, and wherein the resulting nano- and/or micro-particulate formulation comprises d-alpha-tocopheryl polyethylene glycol 1000 succinate at a concentration ranging from about 0.01 wt % to about 20 wt % of the total weight of said formulation.

18. The method of claim 17, wherein d-alpha-tocopheryl polyethylene glycol 1000 succinate is used as an emulsifier during the solvent evaporation/extraction process, and wherein the resulting nano- and/or micro-particulate formulation comprises d-alpha-tocopheryl polyethylene glycol 1000 succinate at a concentration ranging from about 0.01 wt % to about 1 wt % of the total weight of said formulation.

19. The method of claim 17, wherein d-alpha-tocopheryl polyethylene glycol 1000 succinate is used both as an emulsifier during the encapsulation process and as a stabilizer for protecting the at least one pharmacologically active agent and a release modulator for controlling release rate of the at least one pharmacologically active agent, and wherein the resulting nano- and/or micro-particulate formulation comprises d-alpha-tocopheryl polyethylene glycol 1000 succinate at a concentration ranging from about 1 wt % to about 20 wt % of the total weight of said formulation.

20. A composite formulation comprising:
at least a first portion of biocompatible and biodegradable nano- and/or micro-particles loaded with a first pharmacologically active agent and d-alpha-tocopheryl polyethylene glycol 1000 succinate, wherein d-alpha-tocopheryl polyethylene glycol 1000 succinate is present at a first concentration ranging from about 0.01 wt % to about 20 wt % of the total weight of said first portion of nano- and/or micro-particles, and
a second portion of biocompatible and biodegradable nano-particles and/or micro-particles loaded with a second, different pharmacologically active agent and d-alpha-tocopheryl polyethylene glycol 1000 succinate, wherein d-alpha-tocopheryl polyethylene glycol 1000 succinate is present at a second concentration ranging from about 0.01 wt % to about 20 wt % of the total weight of said second portion of nano- and/or micro-particles.

21. The composite formulation of claim 20, wherein the first and second concentrations are different.
